# EUROPÄISCHE PATENTSCHRIFT

(11) **EP 1 190 675 B1**
(45) Veröffentlichungstag und Bekanntmachung des Hinweises auf die Patenterteilung: **28.04.2004**
(21) Anmeldenummer: 00120229.0
(22) Anmeldetag: 26.09.2000
(51) Int. Cl.: A61B 17/15, A61B 17/17

(54) **System zur navigationsgestützten Ausrichtung von Elementen auf einem Körper**
System for navigation-assisted orientation of elements on a body
Système pour l'orientation d'éléments sur un corps assistée par ordinateur

(43) Veröffentlichungstag der Anmeldung: 27.03.2002
(73) Patentinhaber: BrainLAB AG, 85551 Kirchheim/Heimstetten (DE)
(72) Erfinder: Immerz, Martin, 81541 München (DE)
(74) Vertreter: Schwabe - Sandmair - Marx

(56) Entgegenhaltungen:
- WO-A-97/23172
- WO-A-99/15097
- KIENZLE T C ET AL: "TOTAL KNEE REPLACEMENT" IEEE ENGINEERING IN MEDICINE AND BIOLOGY MAGAZINE,US,IEEE INC. NEW YORK, Bd. 14, Nr. 3, 1. Mai 1995 (1995-05-01), Seiten 301-306, XP000505086 ISSN: 0739-5175

## Beschreibung

Die vorliegende Erfindung bezieht sich auf ein insbesondere medizintechnisches System zur navigationsgestützten Ausrichtung von Elementen, insbesondere zur Ausrichtung bzw. Positionierung von Implantaten, welche in Knochen bzw. Gelenke eingesetzt werden, wie zum Beispiel im Bereich des Knies, der Hüfte oder des Rückenmarks.

Es ist bekannt bei der Implantation von künstlichen Kniegelenken Schnittlehren zu verwenden. Dabei werden die Schnittlehren am Femur, dem Oberschenkelknochen, und an der Tibia, dem Schienbein angebracht, wobei die Schnittlehren Schnittebenen der jeweiligen Knochen bestimmen, durch welche die Ausrichtung der femuralen und tibialen Implantate im Wesentlichen festgelegt wird. Dabei sollen die Schnittlehren möglichst so ausgerichtet werden, dass nach Einsetzen der Implantate die mechanische Femur-Achse und die mechanische Tibia-Achse aufeinander ausgerichtet sind.

Aus Kienzle T.C. und anderen "Total Knee Replacement", IEEE ENGINEERING IN MEDICINE AND BIOLOGY, NEW YORK, US, BAND 14, NUMMER 3, 1. MAI 1995, Seiten 301 bis 306 ist ein computerunterstütztes chirurgisches System bekannt, welches einen kalibrierten Roboter verwendet, wobei z.B. die Hüfte eines Patienten in Bezug auf den Roboter festgelegt wird. Der Roboter wird dann verwendet, um die Koordinaten von zwei femuralen Landmark-Pins zu messen. Zur Platzierung eines Schneidblocks am Knochen wird vom Roboter eine Bohrschablone positioniert, woraufhin ein Chirurg Löcher unter Verwendung dieser Bohrschablone bohrt und den Roboter wieder vom Knie entfernt.

Aus der WO 97 / 23172 ist ein computerunterstütztes chirurgisches System bekannt, wobei ein Bein fixiert wird und Datenpunkte mit einem Pointer aufgenommen werden. Eine Koordinatenmessvorrichtung wird verwendet, um eine Schablone bezüglich des Femus zu positionieren.

Dieses dokument wird als nächster Stand der Technik angeschen und umfasst die im unabhängigen Anspruch aufgeführten Merkmale bis auf den Unterschied, dass die Position des Bohrers relativ zur Position des Körpers nicht über die Recheneiheit ermittelt wird und der Bohrer durch den Chirurgen gesteuert wird.

Die WO 99 / 15097 beschreibt eine Vorrichtung und ein Verfahren zur perkutanen Ausrichtung von chirurgischen Implantaten und Instrumenten, um minimal invasive Verfahren durchzuführen. Ein chirurgisches Instrument zur Durchführung eines minimal invasiven Eingriffs zur perkutanen Anordnung von chirurgischen Implantaten wird unter Verwendung eines Navigationssystems relativ zu einem Körperteil positioniert.

Es ist die Aufgabe der vorliegenden Erfindung, ein System zum Anbringen eines Elements an einem Körper vorzuschlagen, mit welchen das Element möglichst exakt positioniert werden kann.

Diese Aufgabe wird durch ein System gemäß dem unabhängigen Anspruch gelöst. Vorteilhafte Ausführungsformen ergeben sich aus den Unteransprüchen.

Mit dem System kann ein Element, wie zum Beispiel eine Schnittlehre bzw. ein Führungselement für eine Schneidvorrichtung, an einem Körper, wie zum Beispiel einem Knochen, dadurch positionsgenau angebracht werden, indem eine Vorrichtung zur Vorbereitung und/oder Herstellung einer Verbindung zwischen dem Element und dem Körper, also zum Beispiel ein Bohrer zum Erzeugen eines Loches oder eine geeignete Befestigungavorrichtung, welche das Element an dem Körper halten kann, so navigiert werden, dass die Verbindungsstellen zwischen dem Körper und dem Element positionsgenau erzeugt werden können. Wird das Element anschließend an den so erzeugten bzw. vorbereiteten Verbindungsstellen angebracht, so ist es automatisch in der gewünschten Position. Das Navigieren der Vorrichtung zur Herstellung oder Vorbereitung einer oder mehrerer Verbindungen bzw. Verbindungsstellen erfolgt bevorzugt dadurch, dass die Position dieser Vorrichtung in Bezug auf die Position des Körpers, an welchem das Element angebracht werden soll, bzw. in Bezug auf eine Soll-Position erfasst wird. Dabei kann die Erfassung der Position sowohl kontinuierlich als auch zu vorgegebenen Zeitpunkten bzw. intermittierend erfolgen. Die erfasste Differenz zwischen Ist-Position und Soll-Position kann entweder ausgegeben, also zum Beispiel auf einem Bildschirm visualisiert werden und/oder unmittelbar für eine automatische Führung verwendet werden.

Es ist also nicht erforderlich die Position eines Elements selbst zu erfassen, um dieses Element in einer gewünschten Position an einem Körper anzubringen, obwohl die Positionserfassung des Elementes natürlich auch durchgeführt werden kann. Die Ausrichtung bzw. Positionierung eines Elements an einem Körper erfolgt durch das positionsgenaue Vorbereiten bzw. Erzeugen der Verbindung bzw. der Verbindungsstellen zwischen dem Element und dem Körper, zum Beispiel durch Navigieren eines Bohrers, wobei sich bei Anbringen des Elements an den vorgesehenen Verbindungsstellen die positionsgenaue Ausrichtung des Elementes ergibt.

Bevorzugt wird die räumliche Struktur des oder der Körper, an welchem bzw. welchen ein oder mehrere Elemente angebracht werden sollen, erfasst. Dies kann zum Beispiel durch Computertomographie, Kernspinresonanz oder andere bekannte Verfahren erfolgen. Dabei ist es möglich den vollständigen Körper zu erfassen, wobei jedoch auch nur interessierende bzw. relevante Teilbereiche des Körpers erfasst werden können. Dabei können dann zum Beispiel durch Interpolation nicht erfasste Bereiche bzw. Strukturen des Körpers ermittelt werden. So kann zum Beispiel nur ein kleiner Bereich der Hüfte von zum Beispiel 5 cm, des Knies von zum Beispiel 10 cm und des Knöchels bzw. oberen Sprunggelenkes von zum Beispiel 5 cm aufeinanderfolgend durch Computertomographie gescannt werden, wobei aus diesen Aufnahmen die für eine genaue Positionierung eines Elementes relevanten Daten ermittelt werden können. Der Abstand zwischen den einzelnen Aufnahmen kann zum Beispiel aus der Bewegung eines Tisches, auf welchem ein zu untersuchendes Bein liegt, gewonnen werden, so dass aus diesen drei Aufnahmen von Teilbereichen die anatomischen Achsen des Beines, d. h. der tatsächliche Verlauf der Knochen und Gelenke, sowie der mechanischen Achsen, d. h. der Verlauf der Traglinien, ermittelt werden können. Allgemein kann nur der für das Anbringen eines Elementes interessierende Bereich eines Körpers erfasst werden, wenn durch geeignete Zusatzinformationen, wie zum Beispiel der relativen Lage der Einzelaufnahmen zueinander, ermittelt werden kann, wie die für das Anbringen des Elementes relevanten Strukturen verlaufen.

Vorteilhaft wird ein Datenmodell des bzw. der Körper, an welchen ein oder mehrere Elemente angebracht werden sollen, erzeugt, um anhand dieses Datenmodells zum Beispiel mit einer geeigneten Software Verfahren zur Verbesserung der Aufnahmequalität oder andere Bildbearbeitungsverfahren, Verfahren zur virtuellen Bearbeitung relevanter Bereiche oder ähnliches durchführen zu können.

Bevorzugt wird eine Mehrzahl von insbesondere dreidimensionalen Datenmodellen eines Implantats oder Einsetzkörpers, also zum Beispiel Datenmodelle verschiedener Implantate, zur Verfügung gestellt. Dies kann zum Beispiel eine Bibliothek mit Implantaten oder Prothesen verschiedener Abmessungen sein, wobei ein oder mehrere Datenmodelle eines oder mehrerer Einsetzkörper ausgewählt werden können.

Vorteilhaft wird insbesondere unter Verwendung der erfassten Daten oder des erzeugten Datenmodells des Körpers ein Implantat oder Einsetzkörper, wie zum Beispiel eine Prothese, virtuell in Relation zu dem Körper positioniert, d. h., dass zum Beispiel ein Knie-Implantat virtuell eingesetzt wird. Das Positionieren kann sowohl automatisch, d. h. zum Beispiel durch einen programmierten Algorithmus erfolgen, wobei das Implantat oder der Einsetzkörper bevorzugt so ausgerichtet wird, dass im eingesetzten Zustand die Hauptbelastungsstellen mit den ermittelten mechanischen Achsen des bzw. der Körper, also zum Beispiel der mechanischen Femur-Achse und der mechanischen Tibia-Achse, zusammenfällt. Es ist auch möglich die Positionierung manuell durchzuführen, d. h. zum Beispiel Daten so auszugeben bzw. anzuzeigen, dass eine Person durch geeignete Eingaben die Positionierung des Einsetzkörpers bzw. der Prothese vornehmen bzw. verändern kann. Bevorzugt wird ein kombiniertes Verfahren durchgeführt, wobei zunächst eine Position von einem geeigneten Algorithmus bzw. einer Software vorgeschlagen wird und anschließend eine Person die Möglichkeit hat, die vorgeschlagene Positionierung zu verändern. Dabei kann zur Feinabstimmung der Einsetzkörper zum Beispiel verschoben, gedreht oder vergrößert bzw. verkleinert werden.

Vorteilhaft wird die gewünschte oder Soll-Position des anzubringenden Elementes aus der Position des Implantats oder Einsetzkörpers ermittelt. Das heißt es kann bei feststehendem Lageverhältnis zwischen Körper und in den Körper einzusetzendem Implantat oder Einsetzkörper bzw. an dem Körper anzubringendem Implantat oder Einsetpzkörper die Position des Elementes bestimmt werden, welches an dem Körper angebracht bzw. eingesetzt werden soll. Wenn zum Beispiel ein künstliches Kniegelenk implantiert werden soll, so steht mit der gewünschten Position des zu implantierenden Kniegelenkes auch die Position einer beispielsweise hierfür verwendeten Schnittlehre fest, welche die Schnittebene an einem Knochen bestimmt, wobei diese Schnittebene als eine Anlagefläche des künstlichen Kniegelenkes dienen kann. Insbesondere ist es vorteilhaft die Position von bestimmten Elementen oder Bereichen der Schnittlehre bzw. des anzubringenden Elementes zu kennen, wie zum Beispiel die Position von Bohrungen in der Schnittlehre, um anhand dieser Daten zum Beispiel das positionsgenaue Bohren von zur Befestigung der Schnittlehre dienenden Löchern in einem Knochen zu steuern bzw. zu überwachen.

Es ist von Vorteil, dass eine Registrierung, d. h. eine klar definierte Ausrichtung des Körpers im Bezug auf ein feststehendes Lage- bzw. Koordinatensystem und/oder die Vorrichtung zur Herstellung oder Vorbereitung der Verbindung zwischen dem Körper und dem anzubringenden Element, zum Beispiel einen Bohrer, und/oder das Element selbst erfolgt. Zur Erfassung der Position des Körpers, zum Beispiel eines oder mehrerer Knochen des Patienten, können verschiedene Verfahren verwendet werden. So können zunächst zum Beispiel Marker fest mit dem Körper verbunden werden, wobei später anhand der Position dieser Marker ermittelt werden kann wie die räumliche Lage des Körpers selbst ist. Die Marker können zum Beispiel ein oder mehrere reflektierende Elemente sein, deren Position von einer oder mehrerer geeigneter Kameras, wie zum Beispiel IR-Kameras, erfasst wird. Als vorteilhaft hat sich die Verwendung von drei Markern erwiesen, welche zum Beispiel in einem gleichschenkligen Dreieck angeordnet sind. Um den Körper, also zum Beispiel einen Knochen, mit daran angebrachten Markern bezüglich des durch zum Beispiel Computertomographie oder Kernspinresonanz ermittelten Datenmodells des Körpers zu registrieren kann beispielsweise ein Vergleich der Oberflächen durchgeführt werden. Dabei können willkürlich eine Anzahl von zum Beispiel 20 Punkten in dem Datenmodell ausgewählt werden, wobei versucht wird eine möglichst genaue Übereinstimmung des Datenmodells mit .dem realen Körper zu erhalten. Weiterhin ist es möglich die Registrierung durch eine oder mehrere Aufnahmen des Körpers mit daran angebrachten Markern vorzunehmen. So kann zum Beispiel mit einem C-Bogen eine oder mehrere Röntgenaufnahme(n) durchgeführt werden, anhand welcher die Registrierung erfolgen kann. Weiterhin besteht die Möglichkeit mit einer Lichtquelle, zum Beispiel einem Laser-Pointer, eine willkürliche Linie auf dem Körper abzufahren, um anhand des von dem Körper reflektierten Lichtes eine Registrierung vorzunehmen.

Ebenso wie die Registrierung des Körpers kann auch der Bohrer bzw. eine geeignete Vorrichtung zur Herstellung oder Vorbereitung einer Verbindung registriert werden. Dazu können auch an dieser Vorrichtung und/oder an dem Führungselement für eine Schneidvorrichtung ein oder mehrere Marker vorgesehen sein, wobei zur Kalibrierung zum Beispiel mit der Bohrspitze auf einen Referenzpunkt gezeigt werden kann, so dass die räumliche Lage von Markern zu Bohrspitze eindeutig bestimmt ist.

Kann nun die Position des Körpers, also zum Beispiel eines Knochens, genau erfasst werden und steht weiterhin fest an welcher Stelle zum Beispiel Bohrungen in dem Knochen angebracht werden sollen, um beispielsweise eine Schnittlehre so zu befestigen, dass ein Schnitt so durchgeführt werden kann, dass ein an diesem Schnitt angebrachtes Implantat wie gewünscht ausgerichtet ist, so kann zum Beispiel ein Bohrer, dessen räumliche Position ebenfalls erfasst wird, so gesteuert werden, dass ein oder mehrere Löcher an den gewünschten Stellen gebohrt werden können. Dabei ist es, wie oben ausgeführt, möglich den Bohrer entweder automatisch zu steuern oder eine Bohrung von Hand vorzunehmen, wobei Abweichungen des Bohrers zum Beispiel bezüglich des Eintrittspunktes und/oder der Bohrachse angezeigt werden können. Weiterhin ist es auch möglich die Bohrtiefe anzugeben, wobei beispielsweise bei erreichter Bohrtiefe eine Warnausgabe erfolgt oder der Bohrer automatisch abgeschaltet wird.

Ergänzend kann durch Fluoroskopie, also das Durchführen von intraoperativen Aufnahmen, eine Überprüfung oder Korrektur der Verfahrensschritte erfolgen.

Ein entsprechendes Computerprogrammprodukt kann direkt in den internen Speicher eines digitalen Computers geladen werden und umfasst Softwarecodeabschnitte, mit welchen ein oder mehrere Schritte des oben beschriebenen Verfahrens ausgeführt werden können, wenn das Produkt auf einem Computer läuft.

Weiterhin kann dieses Computerprogrammprodukt auf einem computergeeigneten Medium gespeichert sein.

Das erfindungsgemäße System zum Anbringen eines Elementes an einem Körper ist im unabhängigen Anspruch definiert. Mit dem erfindungsgemäßen System kann somit eine Lagebestimmung des Bohrers relativ zu dem Körper durchgeführt werden, wodurch zum Beispiel eine automatische Steuerung dieser Vorrichtung oder die Ausgabe von Signalen möglich ist, welche eine mögliche Positionsabweichung anzeigen.

Die Positionserfassungsvorrichtung kann durch eine oder mehrere optische Erfassungselemente, wie zum Beispiel IR-Kameras, oder andere geeignete Erfassungsvorrichtungen realisiert werden, welche zum Beispiel Ultraschall, Funksignale oder andere geeignete Signale erfassen, um die räumliche Position eines Objektes aufzunehmen.

Am Körper und am Bohrer sind ein oder mehrere Marker angebracht, welche von der Positionserfassungsvorrichtung erfasst werden können, so dass eine berührungslose Erfassung der räumlichen Position möglich ist. Es ist jedoch auch denkbar die räumliche Position auf eine andere Art zu erfassen, wie zum Beispiel durch eine Verbindung des zu erfassenden Objektes mit einem Referenzpunkt mittels eines Verbindungselementes, welches durch die Auswertung seiner Krümmung bzw. Biegung einen Rückschluss auf die Lage seines Endpunktes relativ zu seinem Anfangspunkt ermöglicht. Beispielsweise kann ein mehrgelenkiger Arm mit Lagesensoren an den jeweiligen Gelenken oder ein Glasfaserkabel verwendet werden, welches anhand der Brechung bzw. Beugung eines durchgeleiteten Lichtes einen Rückschluss auf seinen räumlichen Verlauf ermöglicht.

Zudem ist eine Recheneinheit vorgesehen, welche zum Beispiel die von der Positionserfassungsvorrichtung erfassten Signale aufnehmen und auswerten kann, um die Registrierung des Objektes mit daran angebrachten Markern bezüglich eines vorher zum Beispiel durch Computertomographie oder Kernspinresonanz aufgenommenen Datenmodells des Körpers durchzuführen. Weiterhin kann die Recheneinheit zur Vorbereitung oder Durchführung eines oder mehrerer der oben beschriebenen Verfahrensschritte dienen.

Bevorzugt ist eine optische Anzeige, zum Beispiel ein Bildschirm vorgesehen, welche mit der Recheneinheit verbunden ist, um Daten bzw. Bilder auszugeben, welche dem jeweiligen Betriebszustand des Systems entsprechen, so dass beispielsweise ein dreidimensionales Modell des Körpers mit darin virtuell positioniertem Element dargestellt werden kann oder eventuelle Positionsabweichungen der Vorrichtung zur Vorbereitung oder Herstellung der Verbindung zwischen Körper und Element angezeigt werden können.

Vorteilhaft ist eine Eingabevorrichtung, wie zum Beispiel eine Tastatur, vorgesehen, wobei die Eingabevorrichtung auch mit der Anzeigevorrichtung zum Beispiel in Form eines Touch-Screens kombiniert werden kann und mit der Recheneinheit verbunden sein kann. Über diese Eingabevorrichtung kann zum Beispiel die virtuelle Positionierung des Elements an dem Körper verändert werden oder die Durchführung eines oder mehrerer der oben beschriebenen Verfahrensschritte ausgelöst und/oder korrigiert bzw. angehalten werden.

Bevorzugt ist eine Datenaufzeichnungsvorrichtung zur Aufzeichnung von Daten beim Betrieb des Systems vorgesehen, um zum Beispiel ein durchgeführtes Verfahren später dokumentieren zu können.

Die Erfindung wird nachfolgend anhand einer bevorzugten Ausführungsform beschrieben werden, wobei:
- Figur 1: die Erfassung von Daten eines Körpers zur Erzeugung eines Datenmodells zeigt;
- Figur 2: das Ermitteln der mechanischen Achsen der erfassten Körper im Datenmodell veranschaulicht, welche zur Positionierung eines Einsetzkörpers relevant sind;
- Figur 3: die virtuelle Positionierung des Einsetzkörpers in dem Datenmodell veranschaulicht;
- Figur 4: die Anzeige der mechanischen Achsen nach virtuellem Positionieren des Einsetzkörpers veranschaulicht;
- Figur 5: eine Adapterklammer mit darauf angeordnetem Referenzstern mit drei Markern zeigt;
- Figur 6: eine Instrumentenkalibrierungsmatrix zeigt;
- Figur 7: eine Referenzelement mit drei Markern zeigt, welches an einem Körper angebracht werden kann;
- Figur 8: schematisch einen Schritt beim Durchführen einer Registrierung zeigt;
- Figur 9: einen Bohrer mit erfindungsgemäß darauf angebrachten Markern zeigt; und
- Figur 10: ein Zielsystem zur Führung des Bohrers zeigt.

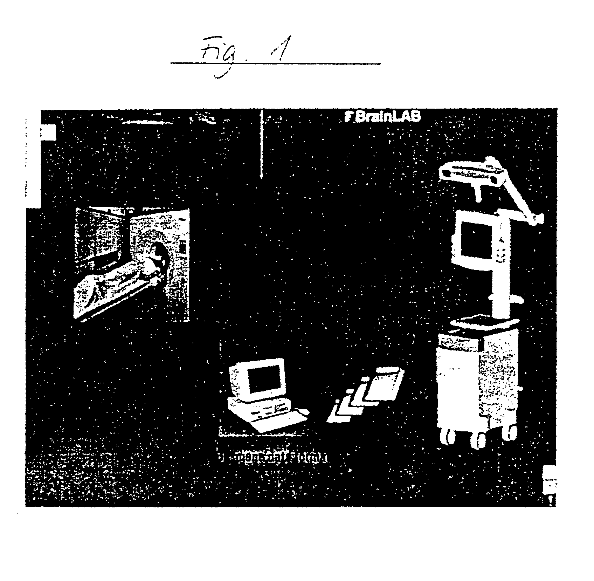

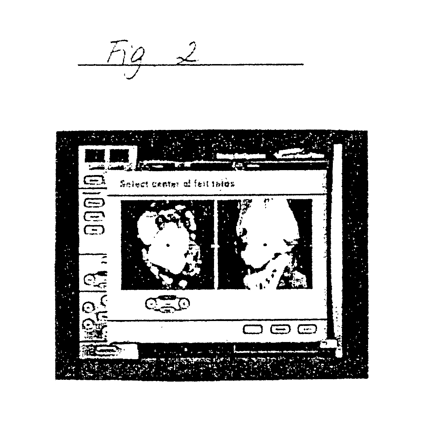

Figur 1 zeigt das Erfassen von Daten, wobei beispielsweise in einem Computertomographen die Hüfte, das Knie und der Knöchel bzw. das Sprunggelenk eines Patienten gescannt werden. Dabei wird bei den jeweiligen Aufnahmen gleichzeitig die Position des Tisches erfasst, auf welchem beispielsweise eine Person liegt, um die relative Lage der einzelnen Aufnahmen zueinander ermitteln zu können. Die aufgenommenen Daten werden zum Beispiel über ein Datennetz oder geeignete Datenträger zu einer Auswerteeinheit übertragen, mit welcher die weitere Bearbeitung der Daten wie in Figur 2 schematisch gezeigt durchgeführt werden kann.

Es wird zum Beispiel durch eine geeignete Software die mechanische Achse von aufgenommenen Körpern ermittelt, zum Beispiel die mechanische Femur-Achse, welche durch den Oberschenkelkopf und den Massenmittelpunkt des Gelenkkopfes definiert werden kann, also derjenigen Teile des Femurs, welche die Tibia bzw. das Schienbein während der Bewegung des Gelenkes berühren bzw. kontaktieren. Weiterhin wird die mechanische Tibia-Achse ermittelt, welche durch den Massenmittelpunkt der proximalen Tibia, also desjenigen Teils des Knochens, welcher näher am Körper liegt, und den Massenmittelpunkt des Thalus bzw. Sprungbeines bestimmt wird. Die ermittelten Massenmittelpunkte können an einem Bildschirm, wie in Figur 2 gezeigt, für beliebige Schnittbilder dargestellt werden.

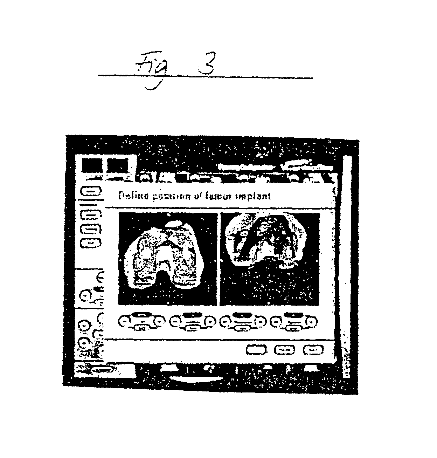

Figur 3 zeigt das virtuelle Festlegen der Position eines Femur-Implantats, welches aus einer gespeicherten Datenbank bzw. Bibliothek aus mehreren Implantaten ausgewählt wurde. Die von einem Algorithmus vorgeschlagene Position des Implantates kann manuell noch verändert, also im virtuellen Modell verschoben bzw. gedreht werden.

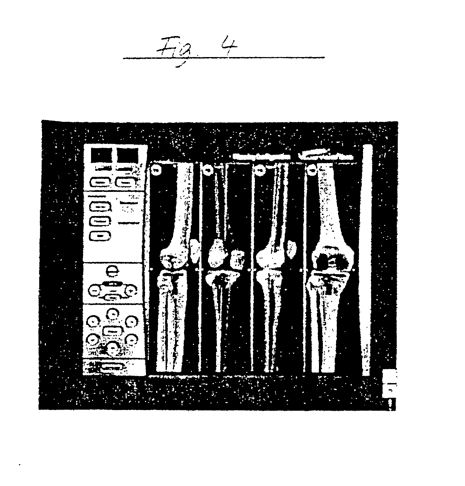

Figur 4 zeigt das Überprüfen der richtigen Position des Implantates, wobei die mechanische Achse bei integriertem Implantat angezeigt wird, um eine präoperative Überprüfung der richtigen Position durchzuführen. Nach Abschluss dieser Überprüfung bzw. gegebenenfalls neuer Positionierung und Überprüfung des richtigen Sitzes des Implantates kann das System verwendet werden.

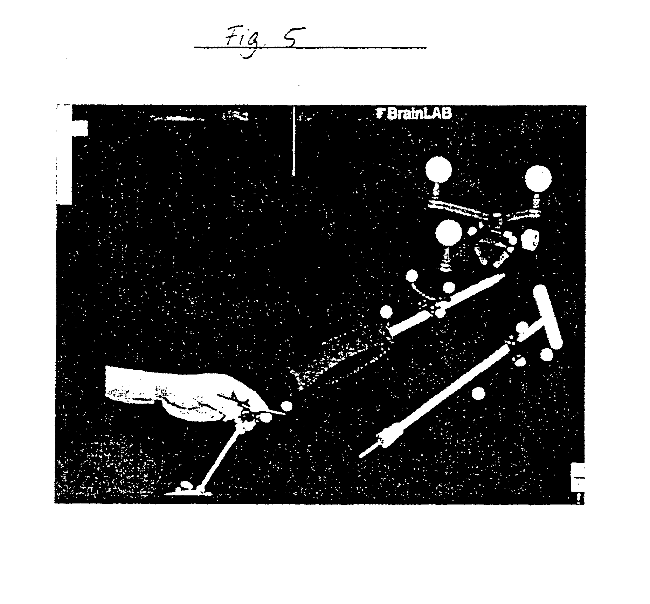

Dazu können Adapterklammern, wie in Figur 5 gezeigt, an beliebigen Instrumenten befestigt werden, um die räumliche Lage dieser Instrumente zu erfassen bzw. eine geeignete Navigation durchzuführen.

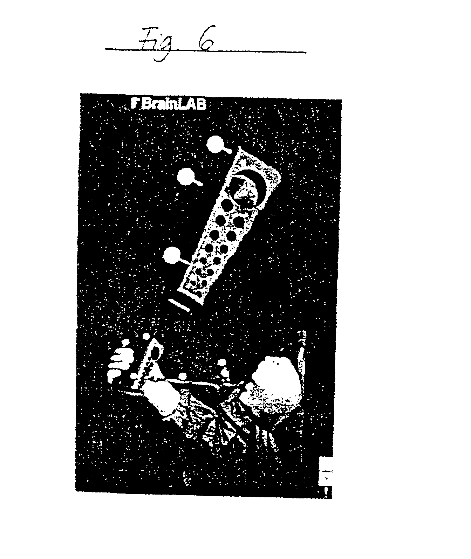

Wie aus Figur 6 ersichtlich kann die Kalibrierung eines Instrumentes mit daran angebrachten Markern mittels einer Kalibrationsmatrix durchgeführt werden, welche ebenfalls mit Markern versehen ist und definierte Bohrungen verschiedener Größe aufweist. Indem die Spitze eines Instrumentes in eine definierte Bohrung der Kalibrationsmatrix gesteckt wird, kann die relative Position der Instrumentenspitze zu den fest mit dem Instrument verbundenen Markern ermittelt werden.

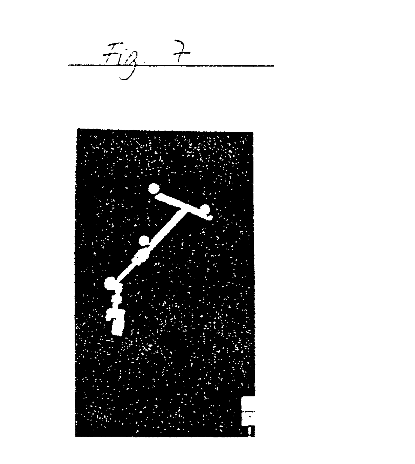

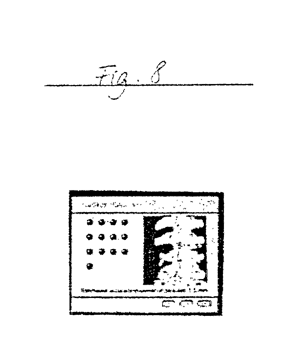

Figur 7 zeigt Referenzelemente, welche an dem Körper, also zum Beispiel dem distalen Femur, d. h. dem entfernteren Teil des Knochens in Bezug auf den Rumpf, und der proximalen Tibia mittels Schrauben befestigt werden können. Diese befestigten Referenzelemente dienen zur genauen Erfassung der räumlichen Lage des Körpers, also zum Beispiel eines oder mehrerer Knochen. Dabei wird, wie in Figur 8 veranschaulicht, eine Registrierung des Körpers zum Beispiel durch surface matching, also den Vergleich bestimmter Referenzpunkte durchgeführt. Dies kann auch durch ein anderes der oben beschriebenen Verfahren erfolgen, wobei in Figur 8 beispielhaft lediglich schematisch das Durchführen einer Registrierung durch Abgleichen der Oberflächen bei verschiedenen Referenzpunkten gezeigt ist.

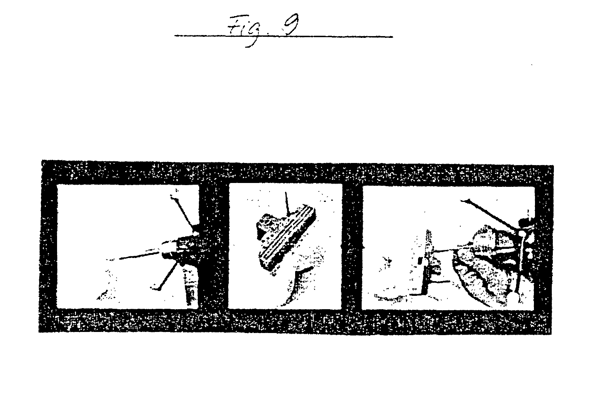

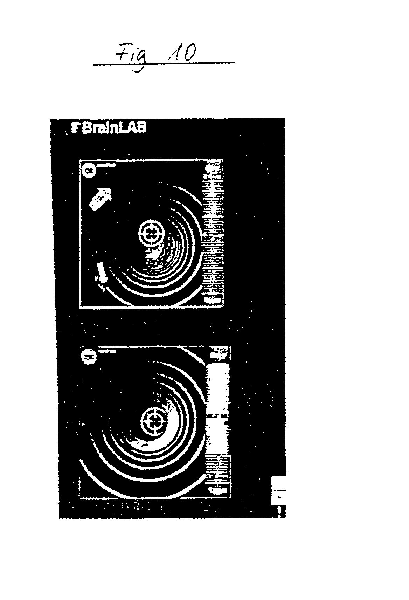

Bei registriertem Körper und virtuell positioniertem und wie in Figur 4 gezeigt bezüglich des korrekten Sitzes überprüftem Implantat kann die Position einer Schnittlehre, und damit die Position der zur Befestigung der Schnittlehre dienenden Löcher ermittelt werden. Ein Bohrer mit einem darauf angebrachten Referenzelement, wie in Figur 5 gezeigt, welcher mit einer Kalibrationsmatrix, wie in Figur 6 gezeigt, kalibriert worden ist, kann somit bezüglich dem registrierten Körper so geführt werden, dass die zur Befestigung der Schnittlehre dienenden Löcher so gebohrt werden, dass ein mit der Schnittlehre durchgeführter Schnitt des Körpers bzw. Knochens zu der gewünschten Position des Implantats an dem Knochen führt. Dabei kann die Führung des Bohrers mit einem in Figur 10 gezeigten Zielsystem durchgeführt werden, wobei an der rechten Seite des Zielsystems die Eindringtiefe dargestellt wird. Links oben gibt ein Pfeil die Winkelabweichung mit Richtung und links unten gibt ein weiterer Pfeil die Positionsabweichung mit Entfernungsangabe an. Mittels des in Figur 10 gezeigten Zielsystems kann die Navigation des in Figur 9 gezeigten Bohrers durchgeführt werden.

## Patentansprüche

1. System zum Anbringen eines Führungselementes für eine Schneidvorrichtung an einem Körper mit:
a) einem Führungselement für eine Schneidvorrichtung;
b) einem Bohrer;
c) eine Positionserfassungsvorrichtung zur Erfassung der Position des Bohrers und zur Erfassung der Position des Körpers;
d) einem Speicher zum Speichern eines dreidimensionalen Datenmodells eines Einsetzkörpers; und
e) einer Recheneinheit zur Vorbereitung und Durchführung eines Verfahrens zum Vorbereiten der und/oder zur Anbringung des Führungselements für eine Schneidvorrichtung an dem Körper, wobei die Position des Bohrers in Bezug auf die Position des Körpers von der Recheneinheit erfasst wird und der Bohrer über die Recheneinheit so navigiert wird, dass Verbindungsstellen so erzeugt werden können, dass das an den so erzeugten Verbindungsstellen angebrachte Führungselement für eine Schneidvorrichtung in einer gewünschten Soll-Position ist, welche aus einer virtuellen Position eines Einsetzkörpers ermittelt wird.

2. System nach dem vorhergehenden Anspruch, wobei die Positionserfassungsvorrichtung optische akustische und/oder Funksignale erfasst.

3. System nach einem der zwei vorhergehenden Ansprüche, wobei ein oder mehrere Marker an dem Führungselement für eine Schneidvorrichtung und/oder dem Körper angebracht sind.

4. System nach einem der drei vorhergehenden Ansprüche, wobei eine Anzeigevorrichtung vorgesehen ist, welche mit der Recheneinheit verbunden ist.

5. System nach einem der vier vorhergehenden Ansprüche, wobei eine Eingabevorrichtung vorgesehen ist, welche mit der Recheneinheit verbunden ist.

6. System nach einem der fünf vorhergehenden Ansprüche, wobei eine Datenaufzeichnungsvorrichtung vorgesehen ist, welche Daten beim Betrieb des Systems aufzeichnen kann.

## Claims

1. A system for applying a guiding element for an incision device to a body, comprising:
a) a guiding element for an incision device;
b) a drill;
c) a position detecting device for detecting the position of said drill and for detecting the position of said body;
d) a memory for storing a three-dimensional data model of an implant; and
e) a computational unit for preparing and performing a method for preparing to and/or for applying said guiding element for an incision device to the body, wherein the position of the drill with respect to the position of the body is detected by said computational unit and the drill is navigated by the computational unit such that connecting points can be produced such that the guiding element for an incision device, applied to the connecting points thus produced, is in a desired position determined from a virtual position of an implant.

2. The system as set forth in the two preceding claim, wherein said position detecting device detects optical, acoustic and/or radio signals.

3. The system as set forth in any one of the preceding two claims, wherein one or more markers are applied to said guiding element for an incision device and/or said body.

4. The system as set forth in any one of the preceding three claims, wherein a display device is provided, connected to said computational unit.

5. The system as set forth in any one of the preceding four claims, wherein an input device is provided, connected to said computational unit.

6. The system as set forth in any one of the preceding five claims, wherein a data recording device is provided which can record data during the operation of said system.

## Revendications

1. Système de mise en place d'un élément de guidage pour un dispositif de coupe sur un corps avec:
a) un élément de guidage pour un dispositif de coupe;
b) un foret;
c) un dispositif d'acquisition de la position du foret et d'acquisition de la position du corps;
d) une mémoire pour mémoriser un modèle de données tridimensionnel d'un corps à insérer; et
e) une unité de calcul pour la préparation et la réalisation d'un procédé de préparation et/ou mettre en place l'élément de guidage pour un dispositif de coupe sur un corps, la position du foret par rapport à la position du corps étant déterminée par l'unité de calcul et le foret étant guidé par l'unité de calcul de telle façon qu'on peut générer des points de raccordement tels que l'élément de guidage fixé aux points de raccordement ainsi générés se trouve dans une position de référence désirée pour un dispositif de coupe, qui est déterminée à partir d'une position virtuelle d'un corps à insérer.

2. Système suivant la revendication précédente, le dispositif d'acquisition de position détectant des signaux optiques, acoustiques et/ou des signaux radio.

3. Système suivant l'une des deux revendications précédentes, un ou plusieurs marqueurs étant fixés sur l'élément de guidage pour un dispositif de coupe et/ou sur le corps.

4. Système suivant l'une des trois revendications précédentes, un dispositif d'affichage étant prévu, lequel est relié à l'unité de calcul.

5. Système suivant l'une des quatre revendications précédentes, un dispositif d'introduction étant prévu, lequel est relié à l'unité de calcul.

6. Système suivant l'une des cinq revendications précédentes, un dispositif d'enregistrement de données étant prévu, lequel peut enregistrer des données pendant le fonctionnement du système.
